(19) Europäisches Patentamt
European Patent Office
Office européen des brevets

(11) Veröffentlichungsnummer : **0 368 227 B1**

(12) **EUROPÄISCHE PATENTSCHRIFT**

(45) Veröffentlichungstag der Patentschrift : **27.01.93 Patentblatt 93/04**

(21) Anmeldenummer : **89120558.5**

(22) Anmeldetag : **07.11.89**

(51) Int. Cl.$^5$ : **C07C 251/40,** C07C 251/52, C07C 323/47, C07D 213/53, C07D 261/08, C07D 275/02, C07D 307/14, C07D 309/08, C07D 309/22, C07D 309/28, C07D 317/28

# (54) Cyclohexenonoximether, Verfahren zu ihrer Herstellung und ihre Verwendung als Herbizid.

Die Akte enthält technische Angaben, die nach dem Eingang der Anmeldung eingereicht wurden und die nicht in dieser Patentschrift enthalten sind.

(30) Priorität : **11.11.88 DE 3838309**

(43) Veröffentlichungstag der Anmeldung : **16.05.90 Patentblatt 90/20**

(45) Bekanntmachung des Hinweises auf die Patenterteilung : **27.01.93 Patentblatt 93/04**

(84) Benannte Vertragsstaaten : **BE CH DE ES FR GB IT LI NL**

(56) Entgegenhaltungen :
**EP-A- 0 131 875**
**EP-A- 0 133 349**
**EP-A- 0 243 313**
**FR-A- 2 240 915**
**GB-A- 2 125 042**
**GB-A- 2 137 200**

(73) Patentinhaber : **BASF Aktiengesellschaft**
**Carl-Bosch-Strasse 38**
**W-6700 Ludwigshafen (DE)**

(72) Erfinder : **Kast, Juergen, Dr.**
**Kastanienstrasse 24**
**W-6737 Boehl-Iggelheim (DE)**
Erfinder : **Kolassa, Dieter, Dr.**
**Moltkestrasse 8**
**W-6700 Ludwigshafen (DE)**
Erfinder : **Meyer, Norbert, Dr.**
**Dossenheimer Weg 22**
**W-6802 Ladenburg (DE)**
Erfinder : **Schirmer, Ulrich, Dr.**
**Berghalde 79**
**W-6900 Heidelberg (DE)**
Erfinder : **Harreus, Albrecht, Dr.**
**Teichgasse 13**
**W-6700 Ludwigshafen (DE)**
Erfinder : **Wild, Jochen, Dr.**
**An der Marlach 7**
**W-6705 Deidesheim (DE)**
Erfinder : **Westphalen, Karl-Otto, Dr.**
**Mausbergweg 58**
**W-6720 Speyer (DE)**
Erfinder : **Wuerzer, Bruno, Dr.**
**Ruedigerstrasse 13**
**W-6701 Otterstadt (DE)**

**EP 0 368 227 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue herbizid wirksame Cyclohexenonoximether der Formel I

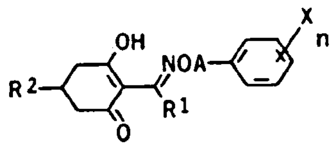

(I)

in der die Substituenten folgende Bedeutung haben:

$R^1$ eine $C_1$-$C_6$-Alkylgruppe;

A $C_4$-Alkyl- oder $C_4$-Alkenylkette, wobei diese Ketten ein bis drei $C_1$-$C_3$-Alkylgruppen und/oder Halogenatome tragen können;

X Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl, Benzyloxycarbonyl und/oder Phenyl, wobei die aromatischen Reste ein bis drei der folgenden Substituenten tragen können: Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkyloxy, Carboxyl, $C_1$-$C_4$-Alkoxycarbonyl- und/oder Benzyloxycarbonyl;

n 0 bis 3 oder 1 bis 5 für den Fall, daß X Halogenatome bedeutet;

$R^2$ eine $C_1$-$C_4$-Alkoxy-$C_1$-$C_6$-alkyl- oder $C_1$-$C_4$-Alkylthio-$C_1$-$C_6$-alkylgruppe;

eine $C_3$-$C_7$-Cycloalkylgruppe oder eine $C_5$-$C_7$-Cycloalkenylgruppe, wobei diese Gruppen ein bis drei der folgenden Substituenten tragen können: $C_1$-$C_4$-Alkyl, $C_1$-$C_4$-Alkoxy, $C_1$-$C_4$-Alkylthio, $C_1$-$C_4$-Halogenalkyl, Hydroxy und/oder Halogen;

ein 5-gliedriger gesättigter Heterocyclus der ein oder zwei Sauerstoff- und/oder Schwefelatome als Heteroatome enthält und der ein bis drei der folgenden Gruppen tragen kann: $C_1$-$C_4$-Alkyl, $C_1$-$C_4$-Alkoxy, $C_1$-$C_4$-Alkylthio und/oder $C_1$-$C_4$-Halogenalkyl,

ein 6- oder 7-gliedriger Heterocyclus, enthaltend ein oder zwei Sauerstoff- und/oder Schwefelatome und gewinschtenfalls bis zu zwei Doppelbindungen, also z.B. auch keine Doppelbindung, wobei dieser Ring ein bis drei der folgenden Substituenten tragen kann: Hydroxy, Halogen, $C_1$-$C_4$-Alkyl, $C_1$-$C_4$-Alkoxy, $C_1$-$C_4$-Alkylthio und/oder $C_1$-$C_4$-Halogenalkyl,

ein 5-gliedriger Heteroaromat, enthaltend ein bis zwei Stickstoffatome und ein Sauerstoffatom oder ein Schewefelatom, wobei dieser Ring ein bis drei der folgenden Substituenten tragen kann: Halogen, Cyano, $C_1$-$C_4$-Alkyl, $C_1$-$C_4$-Alkoxy. $C_1$-$C_4$-Alkylthio, $C_1$-$C_4$-Halogenalkyl, $C_2$-$C_6$-Alkenyl, $C_2$-$C_6$-Alkenyloxy, $C_2$-$C_6$-Halogenalkenyl und/oder $C_1$-$C_4$-Alkoxy-$C_1$-$C_4$-alkyl,

eine Phenylgruppe oder eine Pyridylgruppe, wobei diese Gruppen ein bis drei der folgenden Substituenten tragen können: Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_3$-$C_6$-Alkenyloxy, $C_3$-$C_6$-Alkinyloxy und/oder $-NR^3R^4$, worin

$R^3$ Wasserstoff, ein $C_1$-$C_4$-Alkylgruppe, eine $C_3$-$C_6$-Alkenylgruppe oder eine $C_3$-$C_6$-Alkinylgruppe und

$R^4$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder ein Benzoylrest, wobei der aromatische Ring ein bis drei der folgenden Substituenten tragen kann: Nitro, Cyano, Halogen, $C_1$-$C_4$-Alkyl, $C_1$-$C_4$-Alkoxy, $C_1$-$C_4$-Alkylthio und/oder $C_1$-$C_4$-Halogenalkyl

bedeuten,

sowie ihre landwirtschaftlich nutzbaren Salze und Ester von $C_1$-$C_{10}$-Carbonsäuren und anorganischen Säuren.

Außerdem betrifft die Erfindung ein Verfahren zu ihrer Herstellung sowie ihre Anwendung als Pflanzenschutzmittel.

Die erfindungsgemäßen Cyclohexenone I haben offensichtlich sauren Charakter, d.h. sie können einfache Umsetzungsprodukte wie Salze von Akali- oder Erdalkaliverbindungen oder Enolester bilden.

Die Verbindungen der Formel I können in mehreren tautomeren Formen auftreten, die alle vom Anspruch erfaßt werden.

In der Literatur sind Cyclohexenone der allgemeinen Formel I'

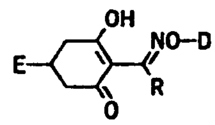

I′

in der u.a.

- D Benzyl und E 2-Ethylthiopropyl (US-A 4 440 566);
- D Benzyl, But-2-enyl und E einen substituierten 5-gliedrigen Heteroarylrest (EP-A 238 021, EP-A 125 094);
- D Benzyl, But-2-enyl und E ein substituiertes Phenyl (EP-A 80 301);
- D But-2-enyl und E einen 5- bis 7-gliedrigen heterocyclischen Ring mit bis zu zwei O-, S-Atomen und mit bis zu zwei Doppelbindungen (EP-A 218 233)

bedeutet, als Herbizide beschrieben.

Insbesondere sind aus der zur U.S. 4,440,566 äquivalenten GB-A 2 125 042 herbizid wirksame Cyclohexan-1,3-dione I″ bekannt,

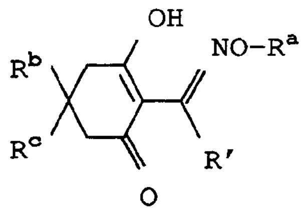

I″

wobei R′ $C_1$-$C_6$-Alkyl oder Phenyl, $R^a$ u.a. $C_6$-$C_{12}$-Halogenaryl oder durch ein bis drei Halogen-, $C_1$-$C_4$-Alkyl- oder $C_1$-$C_4$-Halogenalkylreste substituiertes Benzyl und $R^b$ und $R^c$ Wasserstoff, $C_1$-$C_3$-Alkyl, $C_1$-$C_6$-Alkylthio oder $C_2$-$C_8$-Alkylthioalkyl bedeuten.

Des weiteren lehrt die FR-A 2 240 915, daß Cyclohexan-Derivate der Formel I‴

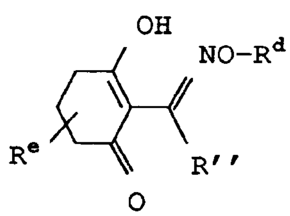

I‴

in der R″ für Wasserstoff, Alkyl oder Phenyl, $R^d$ u.a. für Benzyl und $R^e$ u.a. für bis zu 6 Alkyl-, Alkoxycarbonyl-, Phenyl-, Halogenphenyl-, Methoxyphenyl-, Styryl-, Furanyl- oder Thienylreste stehen, ebenfalls herbizid wirksam sind.

Es werden jedoch Verbindungen gesucht, die bei geringer Aufwandmenge hohe Selektivität aufweisen, d.h. unerwünschte Pflanzen bekämpfen, ohne dabei die Kulturpflanzen zu schädigen.

Entsprechend dieser Aufgabe wurden die neuen Cyclohexenonoximether der Formel I gefunden, die sich durch eine gute herbizide Wirkung gegen unerwünschte Gräser auszeichnen. Die Verbindungen sind mit breitblättrigen Kulturpflanzen sowie einige mit Gramineenkulturen wie Reis verträglich.

Die Cyclohexenone der Formel I können in an sich bekannter Weise aus schon bekannten Derivaten der Formel II (EP-A 80 301, EP-A-125 094, EP-A 142 741, US-A 4 249 937, EP-A 137 174 und EP-A 177 913) und den entsprechenden Hydroxylaminen der Formel III (Houben-Weyl, 10/1 S. 1181 ff) hergestellt werden (EP-A 169 521).

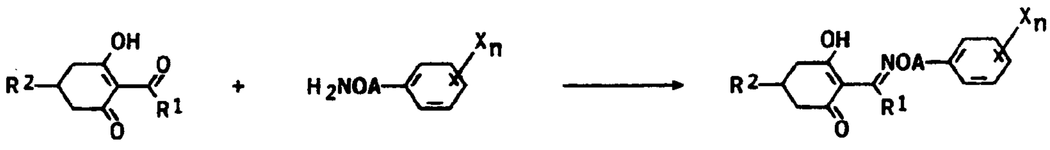

II III I

Zweckmäßig führt man die Umsetzung in heterogener Phase in einem Lösungsmittel, bei einer ausreichenden Temperatur unterhalb von etwa 80°C, in Gegenwart einer Base durch und verwendet das Hydroxylamin III in Form seines Ammoniumsalzes.

Geeignete Basen sind z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent bezogen auf die Ammoniumverbindung zugesetzt.

Als Lösungsmittel eignen sich beispielsweise Dimethylsulfoxid; Alkohole wie Methanol, Ethanol und Isopropanol; aromatische Kohlenwasserstoffe wie Benzol und Toluol; chlorierte Kohlenwasserstoffe wie Chloroform und Dichlorethan; aliphatische Kohlenwasserstoffe wie Hexan und Cyclohexan; Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Die Zielverbindung kann z.B. durch Einengen der Mischung, Verteilung des Rückstandes in Methylenchlorid/Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für die Verbindung II erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol; aliphatische und aromatische, gegebenenfalls chlorierte Kohlenwasserstoffe wie Hexan, Cyclohexan, Methylenchlorid, Toluol und Dichlorethan; Ester wie Essigsäureethylester; Nitrile wie Acetonitril und cyclische Ether wie Dioxan und Tetrahydrofuran.

Alkalimetallsalze der Verbindungen I können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise heigestellt werden, ebenso Ammonium- und Phosphoniumsalze mittels Ammoniak, Phosphonium-, Sulfonium- oder Sulfoxoniumhydroxiden.

Die Verbindungen des Typs II können z.B. aus den entsprechenden Cyclohexan-1,3-dionen der Formel IV

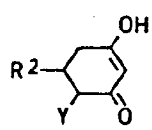

IV,

in der

Y Wasserstoff oder Methoxycarbonyl bedeutet

nach bekannten Methoden (Tetrahedron Lett., 2491 (1975)) hergestellt werden.

Es ist auch möglich, die Verbindungen der Formel II über die Zwischenstufe der Enolester V herzustellen, die bei der Umsetzung von Verbindungen der Formel IV mit Säurechloriden VI in Gegenwart von Basen anfallen und anschließend mit bestimmten Imidazol- oder Pyridinderivaten umgelagert werden (JP-OS 79/063 052).

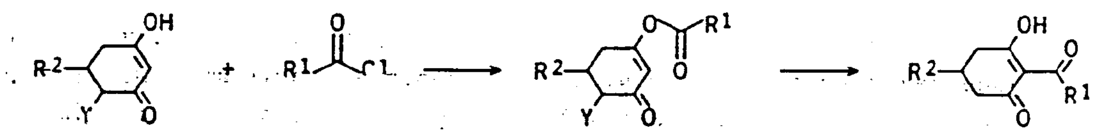

IV VI V II

Zu den Verbindungen der Formel IV gelangt man über eine Reihe bekannter Verfahrensschritte ausgehend von bekannten Vorprodukten.

Die Synthese der Hydroxylamine III in denen A eine substituierte oder unsubstituierte But-2-enylenbrücke bedeutet erfolgt gemäß dem nachstehenden Reaktionsschema ausgehend von Anilinderivaten VII durch Diazotierung, anschließende Kupplung des Diazoniumsalzes mit einem entsprechend substituierten Butadien VIII. Das so erhaltene Gemisch aus IXa und IXb wird mit einem cyclischen Hydroxyimid X gekoppelt und das erhaltene geschützte Hydroxylaminderivat XI mit 2-Aminoethanol zum freien Hydroxylamin III gespalten.

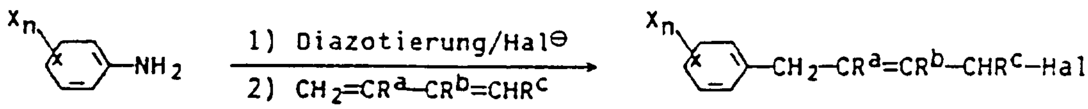


VII VIII IXa

+ 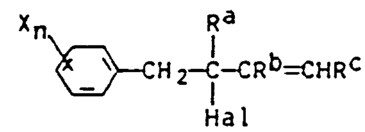 IXb

IXa + IXb + 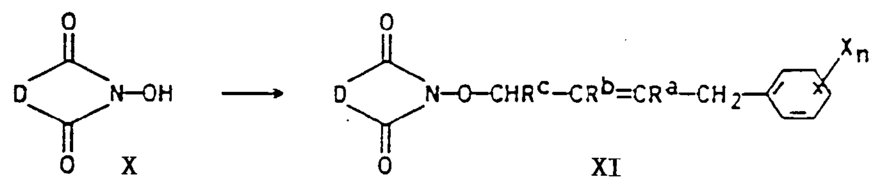

X XI

XI + 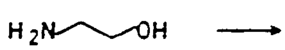 III

Die Reste $R^a$, $R^b$ und $R^c$ bedeuten hierbei unabhängig voneinander Wasserstoff, $C_1$-$C_3$-Alkylgruppen und/oder Halogenatome. Hal bedeutet Halogenatom, vorzugsweise Chloratom.

Als cyclische Hydroxyimide kommen beispielsweise folgende Substanzen in Betracht:

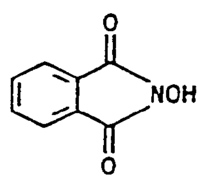 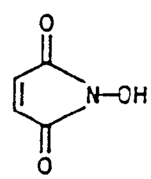 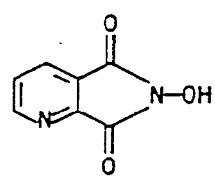

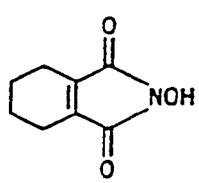 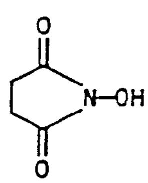 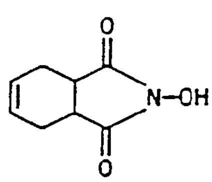

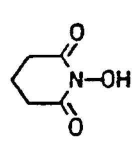

Die zur Synthese der neuen Hydroxylamine der Formel III benötigten Halogenide XIa lassen sich nach literaturbekannten Verfahren im Gemisch mit IXb herstellen, beispielsweise durch die Umsetzung von Diazoniumsalzen aromatischer und heteroaromatischer Aniline mit Dienen. Die Anwendungsbreite der Reaktion ist in Organic Reactions 11 (1960) 189 bzw. 24 (1976) 225 abgehandelt.

Bei der Kopplung der isomeren Halogenide IXa und IXb an ein cyclisches Hydroxyimid der Formel X entstehen ausschließlich die cyclischen Imidether der Formel XI, die nach Abspaltung der Schutzgruppe am Stickstoff die Hydroxylamine III liefern.

Die Umsetzung des Gemisches aus IXa und IXb mit einem Hydroxyimid X erfolgt in Gegenwart eines Säurebindenden Mittels und eines Lösungsmittels. Aus Kostengründen kommt als Hydroxyimid X bevorzugt Hydroxyphthalimid zum Einsatz.

Als säurebindende Mittel eignen sich Alkalimetallcarbonate wie Kalium- oder Natriumcarbonat, Alkalimetallhydrogencarbonate wie Kalium- und Natriumhydrogencarbonat, tertiäre Amine wie Trimethyl- und Triethylamin und basische Heterocyclen wie Pyridin. Aus Kostengründen kommen bevorzugt Kalium- und Natriumcarbonat in Betracht.

Als Lösungsmittel eignen sich aprotisch dipolare organische Lösungsmittel wie z.B. Dimethylformamid, Dimithylsulfoxid und/oder Sulfolan.

Ferner ist auch eine Alkylierung unter Phasentransferbedingungen möglich. Als organische Lösungsmittel werden hierbei mit Wasser nicht mischbare Verbindungen wie Kohlenwasserstoffe oder Chlorkohlenwasserstoffe eingesetzt. Als Phasentransferkatalysatoren eignen sich quartäre Ammonium- und Phosphoniumsalze.

Die Spaltung der cyclischen Imidether XI gelingt analog einem in EP-A 244 786 beschriebenen Verfahren mit Alkanolaminen. Die Hydroxylamine III können nach diesem Verfahren als freie Basen oder nach Fällung mit Säuren als Salze isoliert werden. Gut kristallisierende Salze erhält man durch Umsetzung der Basen mit Oxalsäure.

Im Hinblick auf die biologische Wirksamkeit werden Cyclohexenone der Formel I bevorzugt, in denen die Substituenten folgende Bedeutung haben:

$R^1$ Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Ethyl und Propyl;

A Alkylen oder Alkenylen wie Butylen, But-2-enylen und But-3-enylen, welches ein- bis dreifach durch insbesondere Methyl bzw. Ethyl und/oder Fluor bzw. Chlor substituiert sein kann; bei den ungesättigten Ketten können sowohl die cis- als auch die trans-Form auftreten. Insbesondere bevorzugt ist But-2-enylen.

X Halogen wie Fluor, Chlor, Brom und Iod, insbesondere Fluor und Chlor;

Alkyl wie Methyl, Ethyl, Propyl, 1-Methylethyl, Butyl, 1-Methylpropyl, 2-Methylpropyl und 1,1-Dimethylethyl, insbesondere Methyl und 1,1-Dimethylethyl,

Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy,

Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio,

Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifuormethyl, 2,2,2-Trifluorethyl und Pentafluorethyl,

Halogenalkoxy wie Difluormethoxy, Trifluormethoxy, Chlor-difluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy,

Alkoxycarbonyl wie Methoxycarbonyl, Ethoxycarbonyl, Propyloxycarbonyl, 1-Methylethoxycarbonyl, Butyloxycarbonyl und 1,1-Dimethylethoxycarbonyl , insbesondere Methoxycarbonyl, Ethoxycarbonyl und 1,1-Dimethylethoxycarbonyl, insbesondere Methoxycarbonyl sowie

Nitro, Cyano,

Benzoxycarbonyl und Phenyl, wobei die aromatischen Reste ihrerseits ein bis drei der folgenden Reste tragen können: Nitro, Cyano, Carboxyl, Benzyloxycarbonyl, Halogen wie im allgemeinen und im Besonderen bei X genannt; Alkyl wie bei $R^1$ genannt, insbesondere Methyl, Ethyl und 1-Methylethyl; Alkoxy wie vorstehend genannt, insbesondere Methoxy und Ethoxy; Alkylthio wie vorstehend genannt, insbesondere Methylthio; Halogenalkyl wie vorstehend genannt, insbesondere Trifluormethyl; Halogenalkoxy wie vorstehend genannt, insbesondere Difluormethoxy und Trifluormethoxy und/oder Alkoxycarbonyl wie vorstehend genannt, insbesondere Methoxycarbonyl und Ethoxycarbonyl.

Besonders bevorzugt sind bei diesen aromatischen Resten unsubstituierte oder einfach substituierte Vertreter.

n 0, 1, 2 oder 3, insbesondere 0, 1 und 2. Bei mehreren Resten X können die Substituenten gleich oder verschieden sein.

$R^2$ Alkyl wie unter $R^1$ genannt, welches eine der unter X genannten Alkoxy- oder Alkylthiogruppe bevorzugt in 1-, 2- oder 3-Position tragen kann, insbesondere 2-Ethylthiopropyl;

5-gliedriges Heterocycloalkyl wie Tetrahydrofuranyl, Tetrahydrothienyl, Dioxolanyl, Dithiolanyl und Oxathiolanyl, insbesondere Tetrahydrofuranyl, Tetrahydrothienyl und Dioxolanyl, wobei diese Ringe ein bis drei der bereits unter X genannten $C_1$-$C_4$-Alkylgruppen, $C_1$-$C_4$-Alkoxygruppen, $C_1$-$C_4$-Alkylthiogruppen und/oder $C_1$-$C_4$-Halogenalkylgruppen tragen können,

5-gliedriges Heteroaryl wie Pyrrolyl, Pyrazolyl, Imidazolyl, Isoxazolyl, Oxazolyl, Isothiazolyl, Thiazolyl, Furanyl und Thienyl, insbesondere Isoxazolyl und Furanyl,

ein 6- oder 7-gliedriger Heterocyclus wie Tetrahydropyran-3-yl, Dihydropyran-3-yl, Tetrahydropyran-4-yl, Dihydropyran-4-yl, Tetrahydrothiopyran-3-yl, Dihydrothiopyran-3-yl, Tetrahydrothiopyran-4-yl, Dihydrothiopyran-4-yl und Dioxepan-5-yl, insbesondere Tetrahydropyran-3-yl, Tetrahydropyran-4-yl und Tetrahydrothiopyran-3-yl,

ein Phenyl- oder ein Pyridylrest,

wobei die cyclischen Reste ein bis drei der unter X genannten Alkylgruppen, Alkoxygruppen, Alkylthiogruppen und/oder Halogenalkylgruppen tragen können.

Die 5-gliedrigen Heteroaromaten in der Bedeutung $R^2$ können als Substituenten folgende Reste tragen:

Halogenatom wie unter X genannt, insbesondere Fluor und Chlor,

Alkoxyalkyl wie Methoxymethyl, 2-Methoxyethyl, 2-Methoxypropyl, 3-Methoxypropyl, 2-Methoxy-1-methylethyl, Ethoxymethyl, 2-Ethoxyethyl, 2-Ethoxypropyl, 3-Ethoxypropyl, 2-Ethoxy-1-methylethyl und 1-Ethoxy-1-methylethyl, insbesondere Methoxyethyl und Ethoxyethyl,

Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butnyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere 1-Methylethenyl bzw. entsprechendes Alkenyloxy und/oder Halogenalkenyl.

Die 6- und 7-gliedrigen Heterocyclen können neben den vorstehend genannten Substituenten auch Hydroxygruppen tragen.

Bei den Phenyl- und Pyridylresten kommen als Substituente neben den obengenannten Gruppen auch folgende Reste in Betracht:

Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy, insbesondere 2-Propenyloxy und 2-Butenyloxy;

Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy,

3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, insbesondere 2-Propinyloxy und 2-Butinyloxy;

Amino, welches ein oder zwei der folgenden Reste tragen kann: Alkyl wie bei X genannt, insbesondere Methyl und Ethyl; Alkenyl wie vorstehend genannt, insbesondere 2-Propenyl und 2-Butenyl;

Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, insbesondere 2-Propinyl und 2-Butinyl und/oder

Acyl wie Acetyl, Propionyl, Butyryl, 2-Methylpropionyl, Pentanoyl, 2-Methylbutyryl, 3-Methylbutyryl, 2,2-Dimethylpropionyl, Hexanoyl, 2-Methylpentanoyl, 3-Methylpentanoyl, 4-Methylpentanoyl, 2,2-Dimethylbutyryl, 2,3-Dimethylbutyryl, 3,3-Dimethylbutyryl und 2-Ethylbutyryl, insbesondere Acetyl und Propionyl oder Benzoyl.

Insbesondere bevorzugte Cyclohexenonoximether der Formel I sind in den folgenden Tabellen zusammengefaßt.

Tabelle A

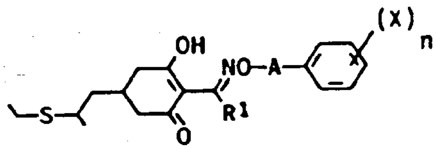

| R1 | A | X |
|---|---|---|
| $CH_2CH_3$ | $(CH_2)_4$ | H |
| $(CH_2)_2CH_3$ | $(CH_2)_4$ | H |
| $CH_2CH_3$ | $(CH_2)_2CH=CH$ | H |
| $(CH_2)_2CH_3$ | $(CH_2)_2CH=CH$ | H |
| $CH_2CH_3$ | $(CH_2)_4$ | 4-$CF_3$ |
| $(CH_2)_2CH_3$ | $(CH_2)_4$ | 4-$CF_3$ |
| $CH_2CH_3$ | $(CH_2)_4$ | 3-F |
| $(CH_2)_2CH_3$ | $(CH_2)_4$ | 3-F |
| $CH_2CH_3$ | $(CH_2)_4$ | 4-F |
| $(CH_2)_2CH_3$ | $(CH_2)_4$ | 4-F |
| $CH_2CH_3$ | $(CH_2)_4$ | 3-$CH_3$ |
| $(CH_2)_2CH_3$ | $(CH_2)_4$ | 3-$CH_3$ |
| $CH_2CH_3$ | $(CH_2)_4$ | 4-$OCH_3$ |
| $(CH_2)_2CH_3$ | $(CH_2)_4$ | 4-$OCH_3$ |
| $CH_2CH_3$ | $(CH_2)_4$ | 3-$NO_2$ |
| $(CH_2)_2CH_3$ | $(CH_2)_4$ | 3-$NO_2$ |
| $CH_2CH_3$ | $(CH_2)_4$ | 3-CN |
| $(CH_2)_2CH_3$ | $(CH_2)_4$ | 3-CN |
| $CH_2CH_3$ | $(CH_2)_4$ | 3-$CO_2CH_3$ |
| $(CH_2)_2CH_3$ | $(CH_2)_4$ | 3-$CO_2CH_3$ |
| $CH_2CH_3$ | $(CH_2)_4$ | 3-$CO_2$Ph |
| $(CH_2)_2CH_3$ | $(CH_2)_4$ | 3-$CO_2$Ph |
| $CH_2CH_3$ | $(CH_2)_4$ | 4-$OCHF_2$ |
| $(CH_2)_2CH_3$ | $(CH_2)_4$ | 4-$OCHF_2$ |
| $CH_2CH_3$ | $(CH_2)_4$ | 3-$CH_3$, 4-Cl |
| $(CH_2)_2CH_3$ | $(CH_2)_4$ | 3-$CH_3$, 4-Cl |

Tabelle B

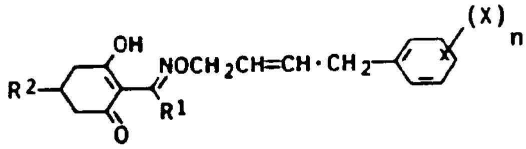

| $R^1$ | $R^2$ | X |
|---|---|---|
| $CH_2CH_3$ | 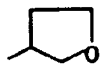 | H |
| $(CH_2)_2CH_3$ | 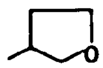 | H |
| $CH_2CH_3$ | 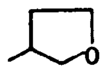 | 4-F |
| $(CH_2)_2CH_3$ | 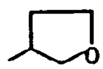 | 4-F |
| $CH_2CH_3$ | 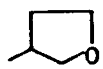 | 3-$CH_3$ |
| $(CH_2)_2CH_3$ | 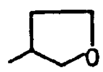 | 3-$CH_3$ |
| $CH_2CH_3$ | 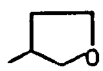 | 3-$CF_3$ |
| $(CH_2)_2CH_3$ | 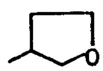 | 3-$CF_3$ |
| $CH_2CH_3$ | 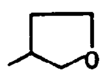 | 4-$C(CH_3)_3$ |
| $(CH_2)_2CH_3$ | 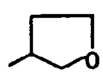 | 4-$C(CH_3)_3$ |
| $CH_2CH_3$ | 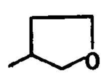 | 3-Cl |
| $(CH_2)_2CH_3$ | 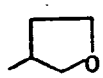 | 3-Cl |

Tabelle B (Fortsetzung)

| $R^1$ | $R^2$ | X |
|---|---|---|
| $CH_2CH_3$ | 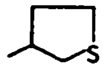 | H |
| $CH_2CH_3$ | 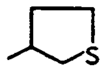 | 4-F |
| $(CH_2)_2CH_3$ | 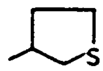 | 4-F |
| $CH_2CH_3$ | 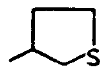 | 3-$CH_3$ |
| $(CH_2)_2CH_3$ | 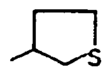 | 3-$CH_3$ |
| $CH_2CH_3$ | 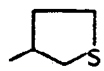 | 3-$CF_3$ |
| $(CH_2)_2CH_3$ | 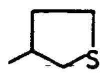 | 3-$CF_3$ |
| $CH_2CH_3$ | 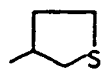 | 4-$C(CH_3)_3$ |
| $(CH_2)_2CH_3$ | 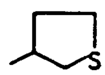 | 4-$C(CH_3)_3$ |
| $CH_2CH_3$ | 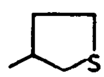 | 3-Cl |
| $(CH_2)_2CH_3$ | 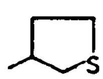 | 3-Cl |
| $CH_2CH_3$ | 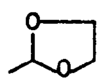 | H |
| $(CH_2)_2CH_3$ | 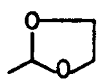 | H |

Tabelle B (Fortsetzung)

| $R^1$ | $R^2$ | X |
|---|---|---|
| $CH_2CH_3$ | 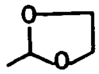 | 4-F |
| $(CH_2)_2CH_3$ | 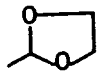 | 4-F |
| $CH_2CH_3$ | 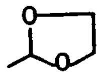 | $3-CH_3$ |
| $(CH_2)_2CH_3$ | 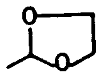 | $3-CH_3$ |
| $CH_2CH_3$ | 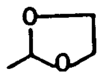 | $3-CF_3$ |
| $(CH_2)_2CH_3$ | 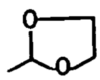 | $3-CF_3$ |
| $CH_2CH_3$ | 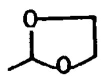 | $4-C(CH_3)_3$ |
| $(CH_2)_2CH_3$ | 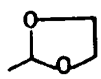 | $4-C(CH_3)_3$ |
| $CH_2CH_3$ | 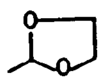 | 3-Cl |
| $(CH_2)_2CH_3$ | 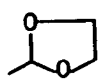 | 3-Cl |
| $CH_2CH_3$ | 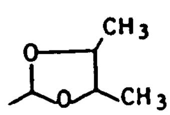 | H |
| $(CH_2)_2CH_3$ | 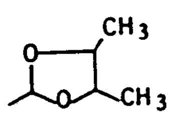 | H |
| $CH_2CH_3$ | 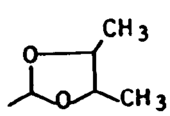 | 4-F |

Tabelle B (Fortsetzung)

| $R^1$ | $R^2$ | X |
|---|---|---|
| $(CH_2)_2CH_3$ | 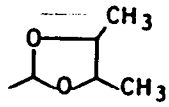 | 4-F |
| $CH_2CH_3$ | 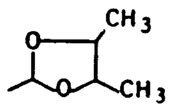 | $3-CH_3$ |
| $(CH_2)_2CH_3$ | 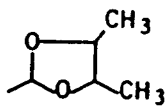 | $3-CH_3$ |
| $CH_2CH_3$ | 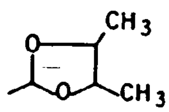 | $3-CF_3$ |
| $(CH_2)_2CH_3$ | 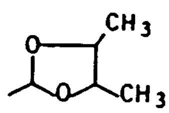 | $3-CF_3$ |
| $CH_2CH_3$ | 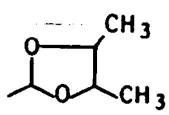 | $4-C(CH_3)_3$ |
| $(CH_2)_2CH_3$ | 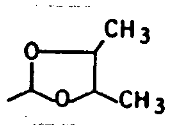 | $4-C(CH_3)_3$ |
| $CH_2CH_3$ | 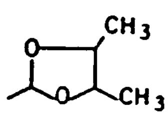 | 3-Cl |
| $(CH_2)_2CH_3$ | 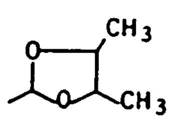 | 3-Cl |
| $CH_2CH_3$ | 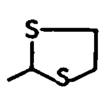 | H |
| $(CH_2)_2CH_3$ | 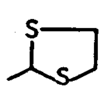 | H |
| $CH_2CH_3$ | 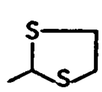 | 4-F |

Tabelle B (Fortsetzung)

| R1 | R2 | X |
|---|---|---|
| $(CH_2)_2CH_3$ | 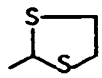 | 4-F |
| $CH_2CH_3$ | 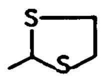 | 3-$CH_3$ |
| $(CH_2)_2CH_3$ | 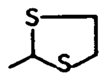 | 3-$CH_3$ |
| $CH_2CH_3$ | 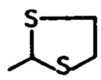 | 3-$CF_3$ |
| $(CH_2)_2CH_3$ | 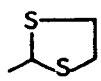 | 3-$CF_3$ |
| $CH_2CH_3$ | 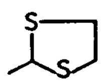 | 4-$C(CH_3)_3$ |
| $(CH_2)_2CH_3$ | 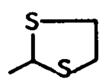 | 4-$C(CH_3)_3$ |
| $CH_2CH_3$ | 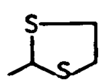 | 3-Cl |
| $(CH_2)_2CH_3$ | 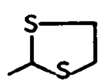 | 3-Cl |
| $CH_2CH_3$ | 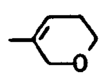 | H |
| $(CH_2)_2CH_3$ | 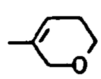 | H |
| $CH_2CH_3$ | 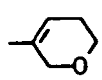 | 4-F |
| $(CH_2)_2CH_3$ | 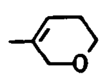 | 4-F |

Tabelle B (Fortsetzung)

| $R^1$ | $R^2$ | X |
|---|---|---|
| $CH_2CH_3$ | 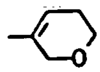 | $3\text{-}CH_3$ |
| $(CH_2)_2CH_3$ | 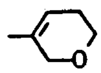 | $3\text{-}CH_3$ |
| $CH_2CH_3$ | 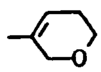 | $3\text{-}CF_3$ |
| $(CH_2)_2CH_3$ | 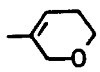 | $3\text{-}CF_3$ |
| $CH_2CH_3$ | 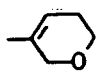 | $4\text{-}C(CH_3)_3$ |
| $(CH_2)_2CH_3$ | 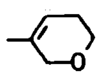 | $4\text{-}C(CH_3)_3$ |
| $CH_2CH_3$ | 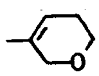 | 3-Cl |
| $(CH_2)_2CH_3$ | 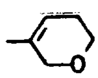 | 3-Cl |
| $CH_2CH_3$ | 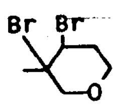 | H |
| $(CH_2)_2CH_3$ | 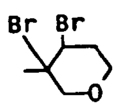 | H |
| $CH_2CH_3$ | 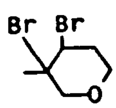 | 4-F |
| $(CH_2)_2CH_3$ | 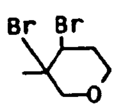 | 4-F |
| $CH_2CH_3$ | 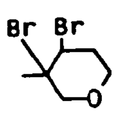 | $3\text{-}CH_3$ |

Tabelle B (Fortsetzung)

| $R^1$ | $R^2$ | X |
|---|---|---|
| $(CH_2)_2CH_3$ | 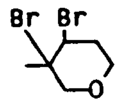 | $3-CH_3$ |
| $CH_2CH_3$ | 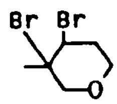 | $3-CF_3$ |
| $(CH_2)_2CH_3$ | 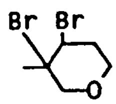 | $3-CF_3$ |
| $CH_2CH_3$ | 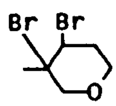 | $4-C(CH_3)_3$ |
| $(CH_2)_2CH_3$ | 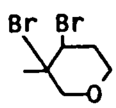 | $4-C(CH_3)_3$ |
| $CH_2CH_3$ | 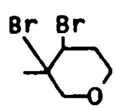 | 3-Cl |
| $(CH_2)_2CH_3$ | 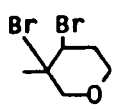 | 3-Cl |
| $CH_2CH_3$ | 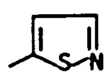 | H |
| $(CH_2)_2CH_3$ | 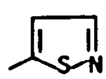 | H |
| $CH_2CH_3$ | 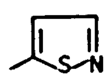 | 4-F |
| $(CH_2)_2CH_3$ | 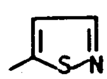 | 4-F |
| $CH_2CH_3$ | 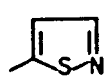 | $3-CH_3$ |

Tabelle B (Fortsetzung)

| R1 | R2 | X |
|---|---|---|
| $(CH_2)_2CH_3$ | 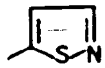 | 3-$CH_3$ |
| $CH_2CH_3$ | 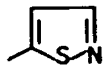 | 3-$CF_3$ |
| $(CH_2)_2CH_3$ | 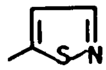 | 3-$CF_3$ |
| $CH_2CH_3$ | 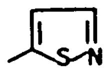 | 4-$C(CH_3)_3$ |
| $(CH_2)_2CH_3$ | 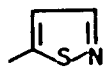 | 4-$C(CH_3)_3$ |
| $CH_2CH_3$ | 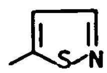 | 3-Cl |
| $(CH_2)_2CH_3$ | 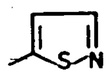 | 3-Cl |
| $CH_2CH_3$ | 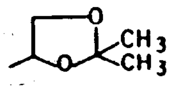 | 3-$CF_3$ |
| $(CH_2)_2CH_3$ | 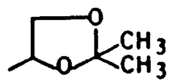 | 3-$CF_3$ |
| $CH_2CH_3$ | 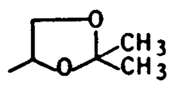 | 4-$C(CH_3)_3$ |
| $(CH_2)_2CH_3$ | 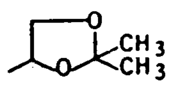 | 4-$C(CH_3)_3$ |
| $CH_2CH_3$ | 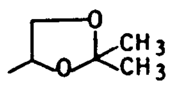 | H |
| $(CH_2)_2CH_3$ | 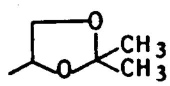 | H |
| $CH_2CH_3$ | 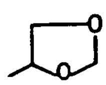 | H |

Tabelle B (Fortsetzung)

| $R^1$ | $R^2$ | X |
|---|---|---|
| $(CH_2)_2CH_3$ | 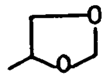 | H |
| $CH_2CH_3$ | 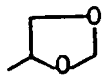 | 3-$CF_3$ |
| $(CH_2)_2CH_3$ | 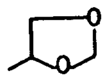 | 3-$CF_3$ |
| $CH_2CH_3$ | 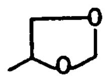 | 4-$C(CH_3)_3$ |
| $(CH_2)_2CH_3$ | 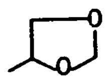 | 4-$C(CH_3)_3$ |
| $CH_2CH_3$ | 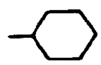 | H |
| $(CH_2)_2CH_3$ | 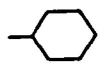 | H |
| $CH_2CH_3$ | 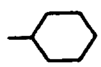 | 3-$CF_3$ |
| $(CH_2)_2CH_3$ | 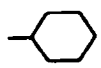 | 3-$CF_3$ |
| $CH_2CH_3$ | 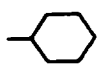 | 4-$C(CH_3)_3$ |
| $(CH_2)_2CH_3$ | 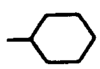 | 4-$C(CH_3)_3$ |
| $CH_2CH_3$ | 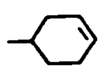 | H |
| $(CH_2)_2CH_3$ | 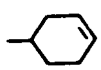 | H |
| $CH_2CH_3$ | 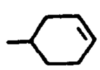 | 3-$CF_3$ |
| $(CH_2)_2CH_3$ | 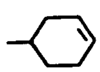 | 3-$CF_3$ |
| $CH_2CH_3$ | 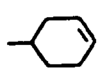 | 4-$C(CH_3)_3$ |
| $(CH_2)_2CH_3$ | 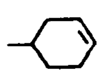 | 4-$C(CH_3)_3$ |

Die Cyclohexenonderivate I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.11 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 2.3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 3.31 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 3.46 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 4.5 werden mit 3 Gewichtsteilen des Natriumsalzes der Diisobutylnaphthalin-$\alpha$-Sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 5.2 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 6.8 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 7.3 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Mittel kann im Vorauflauf- oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,01 bis 3 kg/ha, vorzugsweise 0,05 bis 1,0 kg/ha.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |

| Botanischer Name | Deutscher Name |
|---|---|
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |

| Botanischer Name | Deutscher Name |
|---|---|
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenonderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren, Cyclohexenone, (Hetero)-aryloxypropionsäuren, deren Salze, Ester und Amide und andere in Betracht.

Außerdem kann es von Nutzen sein, die Cyclohexenonderivate der Formel I bzw. sie enthaltende herbide Mittel allein oder in Kombination mit anderen Herbiziden oder auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die im nachstehenden Synthesebeispiel wiedergegebene Vorschrift wurde unter entsprechender Abwandlung der Ausgangsverbindung zur Gewinnung weiterer Verbindungen der Formel I benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

Synthesebeispiel

Herstellvorschrift für Beispiel 3.2

a) Zu einer Lösung von 93,1 g (1 mol) Anilin in 340 ml Wasser und 225 ml konz. Salzsäure gab man bei 0°C 69,1 g (1 mol) Natriumnitrit in 100 ml Wasser.

b) In 840 ml Aceton und 50 ml Wasser wurden bei -15°C 67,6 g (1,25 mol) Butadien eingegast, 15,5 g Kupfer(II)chlorid und 22,5 g Calziumoxid zugegeben und anschließend innerhalb 2 Stunden mit der unter a) hergestellten Diazoniumsalzlösung versetzt. Diese Reaktionsmischung ließ man auf 25°C erwärmen. Nach 6 Stunden Rühren wurde mit Methyl-t-butylether extrahiert, das organische Extrakt eingeengt und über einen Dünnfilmverdampfer (0,2 Torr; 80°C) destilliert. Man erhielt so in 55 % Gesamtausbeute ein Gemisch aus 1-Chlor-4-phenylbut-2-en und 3-Chlor-4-phenylbut-1-en (78:22).

c) In 480 ml trockenes N-Methylpyrrolidon gibt man nacheinander 78,3 g (0,48 mol) N-Hydroxyphthalimid und 44,2 g (0,32 mol) Kaliumcarbonat. Bei 40°C Innentemperatur tropft man 88,8 g (0,54 mol) der nach Beispiel b) erhaltenen Chloridmischung zu. Man erwärmt auf 60°C und rührt weitere 6 Stunden. Nach Abkühlen gießt man auf 2 Liter Eiswasser und filtriert ab. Nach Waschen und Trocknen erhält man 90 % d.Th. an (E)-N-(4-Phenyl-2-butenyloxy)-phthalimid.

Fp.: 70-71°C (Isopropanol)

d) 55,5 g (0,19 mol) des Phthalimidethers e) in 190 ml Essigsäureethylester wurden bei 60°C unter Rühren mit 11,6 g (0,19 mol) Ethanolamin versetzt. Nach 5 Stunden wurde das ausgefallene N-(Hydroxyethyl)-phthalimid abfiltriert und das Filtrat mit 18,8 g Oxalsäure in 30 ml Essigsäureethylester versetzt. Man erhielt so 95 % d.Th. an (E)-4-Phenyl-2-butenyloxy-amin als Oxalat-Salz.

Fp.: 127-129°C.

e) 4,3 g (0,016 mol) 2-Propionyl-5-(3-tetrahydrothiopyranyl)-cyclohexan-1,3-dion, 4,5 g (0,018 mol) 4-Phenyl-but-2-enyloxiammoniumoxalat und 3,0 g Natriumhydrogencarbonat wurden in 100 ml Methanol 16 Stunden bei 25°C gerührt. Das Lösungsmittel wurde bei vermindertem Druck abdestilliert und der Rückstand an Kieselgel mit einem Toluol/Essigsäureethylester-Gemisch im Volumenverhältnis 8:2 chromatographiert. Nach dem Entfernen des Lösungsmittels erhielt man 2,2 g (34,3 % d.Th.) 2-[1-(4-Phenyl-but-2-enyloximino)-propyl]-5-(3-tetrahydrothiopyranyl)-cyclohex-2-en-1-on als Harz.

Soweit in den folgenden Tabellen nichts anderes angegeben ist, liegen die Alkenylreste in der E-Konfiguration vor.

Tabelle 1

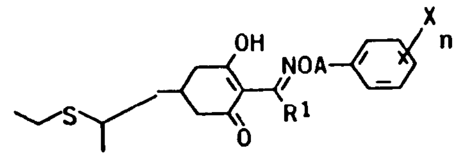

| Nr. | $R^1$ | A | X | n | phys. Daten [Fp (°C); NMR* (δ in ppm)] |
|---|---|---|---|---|---|
| 1.1 | Propyl | But-2-enylen | - | 0 | 4,5 (d, 2H); 5,6-6,1 (m, 2H) |
| 1.2 | Propyl | But-2-enylen | 4-t-Butyl | 1 | 4,5 (d, 2H); 5,6-6,0 (m, 2H); 1,3 (s, 9H) |
| 1.3 | Propyl | But-2-enylen | 4-Trifluormethyl | 1 | 4,5 (d, 2H); 5,6-6,0 (m, 2H) |
| 1.4 | Propyl | But-2-enylen | 4-Fluor | 1 | 4,5 (d, 2H); 5,6-6,0 (m, 2H) |
| 1.5 | Propyl | But-2-enylen | 4-Chlor | 1 | 4,5 (d, 2H); 5,6-6,0 (m, 2H) |
| 1.6 | Propyl | Butylen | - | 0 | 1,0 (s); 4,0-4,2 (m); 7,1-7,4 (m) |
| 1.7 | Ethyl | But-2-enylen | - | 0 | 4,5 (d, 2H); 5,6-6,0 (m, 2H) |
| 1.8 | Propyl | But-2-enylen | 3-Chlor | 1 | 4,5 (d, 2H); 5,6-6,0 (m, 2H) |
| 1.9 | Propyl | But-3-enylen | 4-Chlor | 1 | 4,1 (t, 2H); 6,0-6,5 (m, 2H) |
| 1.10 | Propyl | But-3-enylen | 4-Methyl | 1 | 2,3 (s, 3H); 4,1 (t, 2H); 6,0-6,5 (m, 2H) |
| 1.11 | Propyl | But-3-enylen | 4-Fluor | 1 | 4,1 (t, 2H); 6,0-6,5 (m, 2H) |
| 1.12 | Propyl | But-3-enylen | - | 0 | 4,1 (t, 2H); 6,0-6,6 (m, 2H) |
| 1.13 | Propyl | But-2-enylen | 4-Methoxy | 1 | 3,8 (s, 3H); 4,4 (d, 2H); 5,5-6,0 (m, 2H) |
| 1.14 | Propyl | But-2-enylen | 4-Methyl | 1 | 4,5 (d, 2H); 5,6-6,0 (m, 2H) |

* ausgewählte Signale

Tabelle 2

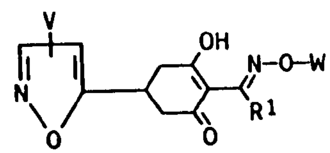

| Nr. | $R^1$ | V | W | phys. Daten [Fp.(°C); NMR* (δ in ppm)] |
|---|---|---|---|---|
| 2.1 | Ethyl | 3-Ethyl | 4-Phenyl-but-2-enyl | 58-59 |
| 2.2 | Propyl | 3-Ethyl | 4-Phenyl-but-2-enyl | 4,5 (d, 2H), 5,6-6,1 (m, 2H) |
| 2.3 | Ethyl | 3-Isopropyl | 4-Phenyl-but-2-enyl | 57-58 |
| 2.4 | Ethyl | 3-(1-Methoxyethyl) | 4-Phenyl-but-2-enyl | 3,3 (s, 3H), 5,6-6,1 (m, 2H) |

Tabelle 3

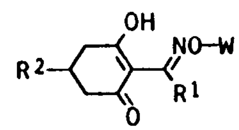

| Nr. | $R^1$ | $R^2$ | W | phys. Daten [Fp (°C); NMR* (δ in ppm)] |
|---|---|---|---|---|
| 3.1 | Propyl | Tetrahydrothiopyran-3-yl | 4-Phenyl-but-2-enyl | 4,5 (d, 2H); 5,5-6,1 (m, 2H) |
| 3.2 | Ethyl | Tetrahydrothiopyran-3-yl | 4-Phenyl-but-2-enyl | 4,5 (d, 2H); 5,6-6,1 (m, 2H) |
| 3.3 | Propyl | Tetrahydrothiopyran-3-yl | 4-(4-Fluorphenyl)-but-2-enyl | 4,5 (d, 2H); 5,6-6,0 (m, 2H) |
| 3.4 | Propyl | Tetrahydrothiopyran-3-yl | 4-(4-Chlorphenyl)-but-2-enyl | 4,5 (d, 2H); 5,6-6,0 (m, 2H) |
| 3.5 | Propyl | Tetrahydrothiopyran-3-yl | 4-(4-Trifluormethylphenyl)-but-2-enyl | 4,5 (d, 2H); 5,6-6,0 (m, 2H) |
| 3.6 | Propyl | Tetrahydrothiopyran-3-yl | 4-(4-t-Butylphenyl)-but-2-enyl | 4,5 (d, 2H); 5,6-6,1 (m, 2H); 1,3 (s, 9H) |
| 3.7 | Ethyl | Tetrahydrothiopyran-3-yl | 4-(4-Fluorphenyl)-but-2-enyl | 4,5 (d, 2H); 5,6-6,0 (m, 2H) |
| 3.8 | Ethyl | Tetrahydrothiopyran-3-yl | 4-(4-Chlorphenyl)-but-2-enyl | 4,5 (d, 2H); 5,6-6,0 (m, 2H) |
| 3.9 | Ethyl | Tetrahydrothiopyran-3-yl | 4-(4-t-Butylphenyl)-but-2-enyl | 4,5 (d, 2H); 5,6-6,0 (m, 2H); 1,3 (s, 9H) |
| 3.10 | Ethyl | Tetrahydrothiopyran-3-yl | 4-(4-Trifluormethylphenyl)-but-2-enyl | 4,5 (d, 2H); 5,6-6,0 (m, 2H) |
| 3.11 | Methyl | Tetrahydrothiopyran-3-yl | 4-Phenyl-but-2-enyl | 4,5 (d, 2H); 5,6-6,0 (m, 2H); 2,4 (s, 3H) |
| 3.12 | Ethyl | Tetrahydropyran-4-yl | 4-Phenyl-but-2-enyl | 62-64 |
| 3.13 | Ethyl | Tetrahydropyran-4-yl | 4-(4-Fluorphenyl)-but-2-enyl | 4,5 (d, 2H); 5,6-6,0 (m, 2H) |

Tabelle 3 (Fortsetzung)

| Nr. | $R^1$ | $R^2$ | W | phys. Daten [Fp. (°C); NMR* (δ in ppm)] |
|---|---|---|---|---|
| 3.14 | Ethyl | Tetrahydropyran-4-yl | 4-(4-Chlorphenyl)-but-2-enyl | 4,5 (d, 2H); 5,6-6,0 (m, 2H) |
| 3.15 | Ethyl | Tetrahydropyran-4-yl | 4-(4-t-Butylphenyl)-but-2-enyl | 99-101 |
| 3.16 | Ethyl | Tetrahydropyran-4-yl | 4-(4-Trifluormethylphenyl)-but-2-enyl | 4,5 (d, 2H), 5,6-6,0 (m, 2H) |
| 3.17 | Propyl | Tetrahydropyran-4-yl | 4-Phenyl-but-2-enyl | 4,5 (d, 2H); 5,6-,61 (m, 2H) |
| 3.18 | Propyl | Tetrahydropyran-4-yl | 4-(4-Fluorphenyl)-but-2-enyl | 4,5 (d, 2H); 5,6-6,0 (m, 2H) |
| 3.19 | Propyl | Tetrahydropyran-4-yl | 4-(4-Chlorphenyl)-but-2-enyl | 4,5 (d, 2H); 5,6-6,0 (m, 2H) |
| 3.20 | Propyl | Tetrahydropyran-4-yl | 4-(4-t-Butylphenyl)-but-2-enyl | 84-86 |
| 3.21 | Propyl | Tetrahydropyran-4-yl | 4-(4-Trifluormethylphenyl)-but-2-enyl | 4,5 (d, 2H); 5,6-6,0 (m, 2H) |
| 3.22 | Ethyl | Tetrahydropyran-3-yl | 4-Phenyl-but-2-enyl | 4,5 (d, 2H); 5,6-6,0 (m, 2H) |
| 3.23 | Ethyl | Tetrahydropyran-3-yl | 4-(4-Fluorphenyl)-but-2-enyl | 4,5 (d, 2H); 5,6-6,0 (m, 2H) |
| 3.24 | Ethyl | Tetrahydropyran-3-yl | 4-(4-Chlorphenyl)-but-2-enyl | 4,5 (d, 2H); 5,6-6,0 (m, 2H) |
| 3.25 | Ethyl | Tetrahydropyran-3-yl | 4-(4-t-Butylphenyl)-but-2-enyl | 4,5 (d, 2H); 5,6-6,0 (m, 2H); 1,3 (s, 9H) |
| 3.26 | Ethyl | Tetrahydropyran-3-yl | 4-(4-Trifluormethylphenyl)-but-2-enyl | 4,5 (d, 2H); 5,6-6,1 (m, 2H) |
| 3.27 | Propyl | Tetrahydropyran-3-yl | 4-Phenyl-but-2-enyl | 4,5 (d, 2H); 5,6-6,0 (m, 2H) |
| 3.28 | Propyl | Tetrahydropyran-3-yl | 4-(4-Fluorphenyl)-but-2-enyl | 4,5 (d, 2H); 5,6-6,0 (m, 2H) |
| 3.29 | Propyl | Tetrahydropyran-3-yl | 4-(4-Chlorphenyl)-but-2-enyl | 4,5 (d, 2H); 5,6-6,0 (m, 2H) |
| 3.30 | Propyl | Tetrahydropyran-3-yl | 4-(4-t-Butylphenyl)-but-2-enyl | 4,5 (d, 2H); 5,6-6,0 (m, 2H); 1,3 (s, 9H) |
| 3.31 | Propyl | Tetrahydropyran-3-yl | 4-(4-Trifluormethylphenyl)-but-2-enyl | 4,5 (d, 2H); 5,6-6,0 (m, 2H) |

Tabelle 3 (Fortsetzung)

| Nr. | $R^1$ | $R^2$ | W | phys. Daten [Fp. (°C); NMR* (δ in ppm)] |
|---|---|---|---|---|
| 3.32 | Ethyl | Tetrahydrothio-pyran-3-yl | 4-(3-Chlorphenyl)-but-2-enyl | 4,5 (d, 2H); 5,6-6,0 (m, 2H) |
| 3.33 | Ethyl | Tetrahydrothio-pyran-3-yl | 4-(4-Methylphenyl)-but-2-enyl | 2,3 (s,3H); 4,5 (d,2H); 5,6-6,0 (m,2H) |
| 3.34 | Ethyl | Tetrahydrothio-pyran-3-yl | 4-(4-Chlorphenyl)-but-3-enyl | 4,2 (t, 2H); 6,1-6,5 (m, 2H) |
| 3.35 | Ethyl | Tetrahydrothio-pyran-3-yl | 4-(4-Methylphenyl)-but-3-enyl | 2,3 (s,3H); 4,2 (t,2H); 6,0-6,5 (m, 2H) |
| 3.36 | Ethyl | Tetrahydrothio-pyran-3-yl | 4-(4-Fluorphenyl)-but-3-enyl | 4,2 (t, 2H); 6,0-6,5 (m, 2H) |
| 3.37 | Ethyl | Tetrahydrothio-pyran-3-yl | 4-Phenylbut-3-enyl | 4,2 (t, 2H); 6,1-6,6 (m, 2H) |
| 3.38 | Propyl | Tetrahydrothio-pyran-3-yl | 4-Phenylbut-3-enyl | 4,2 (t, 2H); 6,1-6,6 (m, 2H) |
| 3.39 | Ethyl | Tetrahydropyran-3-yl | 4-(3-Chlorphenyl)-but-2-enyl | 4,5 (d, 2H); 5,6-6,0 (m, 2H) |
| 3.40 | Ethyl | Tetrahydropyran-3-yl | 4-Phenylbut-3-enyl | 4,2 (t, 2H); 6,1-6,6 (m, 2H) |
| 3.41 | Propyl | Tetrahydropyran-3-yl | 4-Phenylbut-3-enyl | 4,2 (t, 2H); 6,0-6,6 (m, 2H) |
| 3.42 | Ethyl | Tetrahydropyran-4-yl | 4-Phenylbut-3-enyl | 4,2 (t, 2H); 6,1-6,6 (m, 2H) |
| 3.43 | Propyl | Tetrahydropyran-4-yl | 4-Phenylbut-3-enyl | 4,2 (t, 2H); 6,1-6,6 (m, 2H) |
| 3.44 | Propyl | 3,4-Dibromtetrahydro-thiopyran-3-yl | 4-(4-Trifluormethylphenyl)-but-2-enyl | 3,5 (d, 2H); 7,55 (d, 2H) |

Tabelle 3 (Fortsetzung)

| Nr. | $R^1$ | $R^2$ | W | phys. Daten [Fp. (°C); NMR* (δ in ppm)] |
|---|---|---|---|---|
| 3.45 | Propyl | 3,4-Dibromtetrahydropyran-3-yl | 4-(4-Fluorphenyl)-but-3-enyl | 4,15 (t,2H); 5,9-6,2 (m,1H); 6,4 (d,1H) |
| 3.46 | Propyl | 3,4-Dibromtetrahydropyran-3-yl | 4-(4-Fluorphenyl)-but-2-enyl | 4,49 (d, 2H); 5,5-6,1 (m, 2H) |
| 3.47 | Propyl | 3,4-Dibromtetrahydropyran-3-yl | 4-(4-t.-Butylphenyl)-but-2-enyl | 4,49 (d, 2H); 5,55-6,1 (m, 2H) |
| 3.48 | Propyl | 3,4-Dibromtetrahydropyran-3-yl | 4-(4-Methylphenyl)-but-2-enyl | 4,49 (d, 2H); 5,55-6,1 (m, 2H) |
| 3.49 | Propyl | 3,4-Dibromtetrahydropyran-3-yl | 4-(4-Trifluormethylphenyl)-but-2-enyl | 4,52 (s, 2H); 5,5-6,1 (m, 2H) |
| 3.50 | Propyl | 3,4-Dibromtetrahydropyran-3-yl | 4-(3-Chlorphenyl)-but-2-enyl | 4,5 (d, 2H); 5,5-6,1 (m, 2H) |
| 3.51 | Propyl | 3,4-Dibromtetrahydropyran-3-yl | 4-(4-Methylphenyl)-but-3-enyl | 4,18 (t,2H); 6,0-6,2 (m,1H); 6,45 (d,1H) |
| 3.52 | Propyl | 4-Methylcyclohex-3-enyl | 4-(4-Trifluormethylphenyl)-but-2-enyl | 4,05 (m, 2H); 5,5-6,1 (m, 2H) |
| 3.53 | Propyl | 4-Methylcyclohex-3-enyl | 4-(4-Fluormethyl)-but-2-enyl | 4,5 (d, 2H); 5,5-6,1 (m, 2H) |

Tabelle 3 (Fortsetzung)

| Nr. | $R^1$ | $R^2$ | W | phys. Daten [Fp. (°C); NMR* (δ in ppm)] |
|---|---|---|---|---|
| 3.54 | Propyl | Tetrahydrothiopyran--3-yl | 4-(4-Methoxyphenyl)but-2-enyl | 3,8 (s,3H); 4,4 (d,2H); 5,5-6,0 (m,2H) |
| 3.55 | Ethyl | Tetrahydrothiopyran--3-yl | 4-(4-Methoxyphenyl)but-2-enyl | 3,8 (s,3H); 4,5 (d,2H); 5,6-6,0 (m,2H) |
| 3.56 | Propyl | Tetrahydropyran-3-yl | 4-(4-Methoxyphenyl)but-2-enyl | 3,8 (s,3H); 4,5 (d,2H); 5,6-6,0 (m,2H) |
| 3.57 | Ethyl | Tetrahydropyran-3-yl | 4-(4-Methoxyphenyl)but-2-enyl | 3,8 (s,3H); 4,5 (d,2H); 5,6-6,0 (m,2H) |
| 3.58 | Propyl | Tetrahydropyran-4-yl | 4-(4-Methoxyphenyl)but-2-enyl | 3,8 (s,3H); 4,4 (d,2H); 5,5-6,0 (m,2H) |
| 3.59 | Ethyl | Tetrahydropyran-4-yl | 4-(4-Methoxyphenyl)but-2-enyl | 3,8 (s,3H); 4,5 (d,2H); 5,5-6,0 (m,2H) |
| 3.60 | Ethyl | Tetrahydrothiopyran--3-yl | Z-4-Phenylbut-2-enyl | 4,7 (d,2H); 5,8-6,0 (m,2H) |
| 3.61 | Propyl | Tetrahydrothiopyran--3-yl | Z-4-Phenylbut-2-enyl | 4,7 (d,2H); 5,8-6,0 (m,2H) |
| 3.62 | Propyl | Tetrahydropyran-3-yl | Z-4-Phenylbut-2-enyl | 4,7 (d,2H); 5,8-6,0 (m,2H) |
| 3.63 | Propyl | Tetrahydropyran-4-yl | Z-4-Phenylbut-2-enyl | 4,7 (d,2H); 5,8-6,0 (m,2H) |
| 3.64 | Ethyl | Tetrahydropyran-4-yl | Z-4-Phenylbut-2-enyl | 4,7 (d,2H); 5,8-6,0 (m,2H) |
| 3.65 | Propyl | Tetrahydrothiopyran--3-yl | 4-(4-Methylphenyl)but-2-enyl | 2,3 (s,3H); 4,5 (d,2H); 5,6-6,0 (m,2H) |
| 3.66 | Propyl | Tetrahydrothiopyran--3-yl | 4-(3-Chlorphenyl)but-2-enyl | 4,5 (d,2H); 5,6-6,0 (m,2H) |
| 3.67 | Propyl | Tetrahydrothiopyran--3-yl | 4-(3,4-Dichlorphenyl)but--2-enyl | 4,5 (d,2H); 5,6-6,0 (m,2H) |
| 3.68 | Ethyl | Tetrahydrothiopyran--3-yl | 4-(3,4-Dichlorphenyl)but--2-enyl | 4,5 (d,2H); 5,6-6,0 (m,2H) |
| 3.69 | Propyl | Tetrahydropyran-3-yl | 4-(3,4-Dichlorphenyl)but--2-enyl | 4,5 (d,2H); 5,6-6,0 (m,2H) |

Tabelle 3 (Fortsetzung)

| Nr. | $R^1$ | $R^2$ | W | phys. Daten [Fp. (°C); NMR* (δ in ppm)] |
|---|---|---|---|---|
| 3.70 | Propyl | Tetrahydropyran-4-yl | 4-(3,4-Dichlorphenyl)but--2-enyl | 4,5 (d,2H); 5,6-6,0 (m,2H) |
| 3.71 | Propyl | Tetrahydrothiopyran--3-yl | 4-(4-Bromphenyl)but-3-enyl | 4,2 (t,2H); 6,2 (dt,1H); 6,4 (d,1H) |
| 3.72 | Ethyl | Tetrahydrothiopyran--3-yl | 4-(4-Bromphenyl)but-3-enyl | 4,2 (t,2H); 6,2 (dt,1H); 6,4 (d,1H) |
| 3.73 | Propyl | Tetrahydrothiopyran--3-yl | 4-(4-Fluorphenyl)but-3-enyl | 4,6 (t,2H); 6,1 (dt,1H); 6,6 (d,1H) |
| 3.74 | Propyl | Tetrahydrothiopyran--3-yl | 4-(4-Chlorphenyl)but-3-enyl | 4,2 (t,2H; 6,1 - 6,5 (2m,2H) |
| 3.75 | Propyl | Tetrahydrothiopyran--3-yl | 4-(4-Methylphenyl)but-3-enyl | 4,2 (t,2H); 6,1 - 6,2 (m,2H) ; 6,6 (d,1H) |
| 3.76 | Propyl | Tetrahydrothiopyran--3-yl | 4-(4-Bromphenyl) but-3-enyl | 4,2 (t,2H); 6,0-6,4 (2m,2H) |
| 3.77 | Propyl | Tetrahydrothiopyran--4-yl | 4-(4-Bromphenyl) but-3-enyl | 4,2 (t,2H); 6,0-6,5 (2m,2H) |
| 3.78 | Ethyl | Tetrahydrothiopyran--4-yl | 4-(4-Bromphenyl) but-3)enyl | 4,2 (t,2H); 6,0-6,5 (2m,2H) |

Tabelle 4

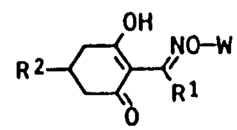

| Nr. | $R^1$ | $R^2$ | W | phys. Daten [Fp (°C); NMR* (δ in ppm)] |
|---|---|---|---|---|
| 4.1 | Ethyl | Phenyl | 4-Phenyl-but-2-enyl | 4,5 (d, 2H); 5,6-6,0 (m, 2H) |
| 4.2 | Propyl | Phenyl | 4-Phenyl-but-2-enyl | 4,5 (d, 2H); 5,6-6,0 (m, 2H) |
| 4.3 | Ethyl | 4-Trifluormethylphenyl | 4-Phenyl-but-2-enyl | 4,5 (d, 2H); 5,6-6,1 (m, 2H) |
| 4.4 | Propyl | 4-Trifluormethylphenyl | 4-Phenyl-but-2-enyl | 56-57 |
| 4.5 | Ethyl | 4-Ethylphenyl | 4-Phenyl-but-2-enyl | 4,5 (d, 2H); 5,6-6,1 (m, 2H) |
| 4.6 | Ethyl | 4-N-Benzoylaminophenyl | 4-Phenyl-but-2-enyl | 103-105 |
| 4.7 | Propyl | 4-Ethoxyphenyl | 4-Phenyl-but-2-enyl | 4,6 (d, 2H); 5,6-6,1 (m, 2H) |
| 4.8 | Propyl | 3-Fluor-4-(N-benzoyl-amino)-phenyl | 4-Phenyl-but-2-enyl | 87-90 |
| 4.9 | Ethyl | 4-Pyridyl | 4-Phenyl-but-2-enyl | 4,5 (d,2H); 5,6-6,2 (m,2H) |
| 4.10 | Propyl | 3-Pyridyl | 4-Phenyl-but-2-enyl | 4,6 (d,2H); 5,6-6,2 (m,2H) |
| 4.11 | Ethyl | 4-(Propinyl-3-oxy)phenyl | 4-Phenyl-but-2-enyl | 4,6 (d,2H); 5,6-6,1 (m,2H) |
| 4.12 | Propyl | 4-Ethylphenyl | 4-Phenyl-but-2-enyl | 4,5 (d,2H); 5,6-6,1 (m,2H) |

Tabelle 5

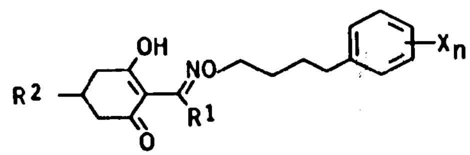

| Nr. | $R^1$ | $R^2$ | $X_n$ | phys. Daten [Fp (°C); NMR* (δ in ppm)] |
|---|---|---|---|---|
| 5.1 | Ethyl | Tetrahydropyran-3-yl | H | 1,12 (t); 7,1-7,4 (m) |
| 5.2 | Propyl | Tetrahydropyran-4-yl | H | 0,99 (t); 7,1-7,4 (m) |
| 5.3 | Methyl | Tetrahydrothiopyran-3-yl | H | 2,31 (s); 3,9-4,1 (m); 7,1-7,35 (m) |
| 5.4 | Ethyl | Tetrahydrothiopyran-3-yl | H | 1,1 (t); 3,9-4,1 (m); 7,1-7,4 (m) |
| 5.5 | Propyl | Tetrahydrothiopyran-3-yl | H | 0,95 (t); 3,9-4,1 (m); 7,1-7,4 (m) |
| 5.6 | Propyl | 4-Methylcyclohex-3-enyl | H | 4,5 (d, 2H); 5,6-6,0 (m, 2H) |
| 5.7 | Methyl | Tetrahydropyran-3-yl | H | |
| 5.8 | Propyl | Tetrahydropyran-3-yl | H | |
| 5.9 | Methyl | Tetrahydropyran-4-yl | H | |
| 5.10 | Ethyl | Tetrahydropyran-3-yl | 4-t-Butyl | |
| 5.11 | Propyl | Tetrahydropyran-3-yl | 4-t-Butyl | |
| 5.12 | Ethyl | Tetrahydrothiopyran-3-yl | 4-t-Butyl | |
| 5.13 | Propyl | Tetrahydrothiopyran-3-yl | 4-t-Butyl | |
| 5.14 | Ethyl | Tetrahydropyran-4-yl | 4-t-Butyl | |
| 5.15 | Propyl | Tetrahydropyran-4-yl | 4-t-Butyl | |

Tabelle 5 (Fortsetzung)

| Nr. | $R^1$ | $R^2$ | $X_n$ | phys. Daten [Fp (°C); NMR* (δ in ppm)] |
|---|---|---|---|---|
| 5.16 | Ethyl | Tetrahydropyran-3-yl | 4-$CF_3$ | |
| 5.17 | Propyl | Tetrahydropyran-3-yl | 4-$CF_3$ | |
| 5.18 | Ethyl | Tetrahydropyran-4-yl | 4-$CF_3$ | |
| 5.19 | Propyl | Tetrahydropyran-4-yl | 4-$CF_3$ | |
| 5.20 | Ethyl | Tetrahydrothiopyran-3-yl | 4-$CF_3$ | |
| 5.21 | Propyl | Tetrahydrothiopyran-3-yl | 4-$CF_3$ | |
| 5.22 | Ethyl | Tetrahydropyran-4-yl | H | |

Tabelle 6

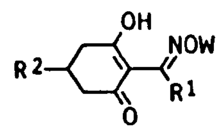

| Nr. | $R^1$ | $R^2$ | W | phys. Daten [Fp (°C); NMR* (δ in ppm)] |
|---|---|---|---|---|
| 6.1 | Ethyl | 3,4-Dihydroxycyclohexyl | 4-(4-Chlorphenyl)-but-2-enyl | 4,4-4,6 (m,2H); 5,5-6,0 (m,2H) |
| 6.2 | Ethyl | 2-Ethylthio-2-methylpropyl | 4-(4-Fluorphenyl)-but-2-enyl | 1,3 (s,6H); 4,5 (d,2H); 5,6-6,0 (m,2H) |
| 6.3 | Ethyl | 2-Ethylthio-2-methylpropyl | 4-Phenylbut-2-enyl | 1,3 (s,6H); 4,5 (d,2H); 5,5-6,1 (m,2H) |
| 6.4 | Ethyl | 2-Ethylthio-2-methylpropyl | 4-Phenylbut-3-enyl | 1,3 (s,6H); 4,2 (t,2H); 6,2 (dt,1H); 6,4 (dt,1H) |
| 6.5 | Ethyl | 2-Ethylthio-2-methylpropyl | 4-(4-Fluorphenyl)-but-3-enyl | 1,3 (s,6H); 4,2 (t,2H); 6,1 (dt,1H); 6,4 (dt,1H) |
| 6.6 | Ethyl | 2-Ethylthio-2-methylpropyl | 4-(4-Chlorphenyl)-but-3-enyl | 1,3 (s,6H); 4,2 (t,2H); 6,2 (dt,1H); 6,5 (dt,1H) |
| 6.7 | Ethyl | 2,6,6-Trimethylcyclohexy--1-enyl | 4-Phenylbut-2-enyl | 1,0 (s,6H); 4,5 (d,2H); 5,8-6,2 (m,2H) |
| 6.8 | Propyl | 2,6,6-Trimethylcyclohexy--1-enyl | 4-Phenylbut-2-enyl | 1,0 (s,6H); 4,5 (d,2H); 5,6-6,1 (m,2H) |

Tabelle 7

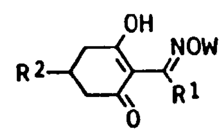

| Nr. | $R^1$ | $R^2$ | W | phys. Daten [Fp (°C); NMR* (δ in ppm)] |
|---|---|---|---|---|
| 7.1 | Propyl | Tetrahydrothiopyran-3-yl | 4-(4-Fluorphenyl)-pent-3-enyl | 2,0 (s,3H); 4,1 (t,2H); 5,7 (t,1H) |
| 7.2 | Ethyl | Tetrahydrothiopyran-3-yl | 4-(4-Fluorphenyl)-pent-3-enyl | 2,0 (s,3H); 4,1 (t,2H); 5,7 (t,1H) |
| 7.3 | Propyl | Tetrahydropyran-3-yl | 4-(4-Fluorphenyl)-pent-3-enyl | 2,0 (s,3H); 4,1 (t,2H); 5,7 (t,1H) |
| 7.4 | Propyl | Tetrahydropyran-4-yl | 4-(4-Fluorphenyl)-pent-3-enyl | 2,0 (s,3H); 4,1 (t,2H); 5,7 (t,1H) |
| 7.5 | Ethyl | Tetrahydropyran-4-yl | 4-(4-Fluorphenyl)-pent-3-enyl | 2,0 (s,3H); 4,1 (t,2H); 5,7 (t,1H) |

Anwendungsbeispiele

Die Wirkung der Cyclohexenonderivate der Formel I auf das Pflanzenwachstum läßt sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmenge für die Vorauflaufanwendung betrug 0,5 kg/ha a.S.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,06, 0,125 und 0,25 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Digitaria sanguinalis | Blutfingerhirse |
| Echinochloa crus-galli | Hühnerhirse |
| Leptochloa fascicularis | - |
| Medicago sativa | Lucerne |
| Oryza sativa | Reis |
| Setaria faberii | Borstenhirse |
| Setaria italica | Ital. Raygras |
| Setaria viridis | Grüne Borstenhirse |

Zur Bekämpfung grasartiger unerwünschter Pflanzen eignen sich beispielsweise die Verbindungen Nr. 3.3, 3.4, 3.8, 3,13, 3,34, 3,36 und 3,73 bei Nachauflaufanwendung von 0,06, 0,125 und 0,25 kg/ha a.S., bei gleichzeitiger guter Verträglichkeit für Reis bzw. Lucerne.

## Patentansprüche

1. Cyclohexenonoximether der allgemeinen Formel I

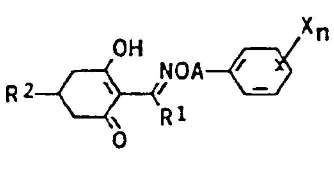

(I)

in der die Substituenten folgende Bedeutung haben:

$R^1$ eine $C_1$-$C_6$-Alkylgruppe;

A eine $C_4$-Alkylen- oder $C_4$-Alkenylenkette, wobei diese Ketten ein bis drei $C_1$-$C_3$-Alkylgruppen und/oder Halogenatome tragen können;

X Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkoxy, Carboxyl, $C_1$-$C_4$-Alkoxycarbonyl, Benzyloxycarbonyl und/oder Phenyl, wobei die aromatischen Reste ein bis drei der folgenden Substituenten tragen können: Nitro, Cyano, Halogen, $C_1$-$C_4$-Alkyl, $C_1$-$C_4$-Alkoxy, $C_1$-$C_4$-Alkylthio, $C_1$-$C_4$-Halogenalkyl, $C_1$-$C_4$-Halogenalkyloxy, Carboxyl, $C_1$-$C_4$-Alkoxycar-

bonyl und/oder Benzyloxycarbonyl;

n 0 bis 3 oder 1 bis 5 für den Fall, daß X Halogen bedeutet;

$R^2$ eine $C_1$-$C_4$-Alkoxy-$C_1$-$C_6$-alkyl- oder $C_1$-$C_4$-Alkylthio-$C_1$-$C_6$-alkylgruppe;

eine $C_3$-$C_7$-Cycloalkylgruppe oder eine $C_5$-$C_7$-Cycloalkenylgruppe, wobei diese Gruppen ein bis drei der folgenden Substituenten tragen können: $C_1$-$C_4$-Alkyl, $C_1$-$C_4$-Alkoxy, $C_1$-$C_4$-Alkylthio, $C_1$-$C_4$-Halogenalkyl, Hydroxy und/oder Halogen;

ein 5-gliedriger gesättigter Heterocyclus der ein oder zwei Sauerstoff- und/oder Schwefelatome als Heteroatome enthält, und der bis zu drei der folgenden Gruppen tragen kann: $C_1$-$C_4$-Alkyl, $C_1$-$C_4$-Alkoxy, $C_1$-$C_4$-Alkylthio und/oder $C_1$-$C_4$-Halogenalkyl;

ein 6- oder 7-gliedriger Heterocyclus, enthaltend ein oder zwei Sauerstoff- und/oder Schwefelatome und bis zu zwei Doppelbindungen, wobei dieser Ring ein bis drei der folgenden Substituenten tragen kann: Hydroxy, Halogen, $C_1$-$C_4$-Alkyl, $C_1$-$C_4$-Alkoxy, $C_1$-$C_4$-Alkylthio, und/oder $C_1$-$C_4$-Halogenalkyl,

ein 5-gliedriger Heteroaromat, enthaltend ein bis zwei Stickstoffatome und ein Sauerstoffatom oder ein Schwefelatom, wobei dieser Ring ein bis drei der folgenden Substituenten tragen kann: Halogen, Cyano, $C_1$-$C_4$-Alkyl, $C_1$-$C_4$-Alkoxy, $C_1$-$C_4$-Alkylthio, $C_1$-$C_4$-Halogenalkyl, $C_2$-$C_6$-Alkenyl, $C_2$-$C_6$-Alkenyloxy, $C_2$-$C_6$-Halogenalkenyl und/oder $C_1$-$C_4$-Alkoxy-$C_1$-$C_4$-alkyl;

eine Phenylgruppe oder eine Pyridylgruppe, wobei diese Gruppen ein bis drei der folgenden Substituenten tragen können: Halogen, Nitro, Cyano, $C_1$-$C_4$-Alkyl, $C_1$-$C_4$-Alkoxy, $C_1$-$C_4$-Alkylthio, $C_1$-$C_4$-Halogenalkyl, $C_3$-$C_6$-Alkenyloxy, $C_3$-$C_6$-Alkinyloxy und/oder $-NR^3R^4$, worin

$R^3$ Wasserstoff, eine $C_1$-$C_4$-Alkylgruppe, eine $C_3$-$C_6$-Alkenylgruppe oder eine $C_3$-$C_6$-Alkinylgruppe und

$R^4$ Wasserstoff, eine $C_1$-$C_4$-Alkylgruppe, eine $C_3$-$C_6$-Alkenylgruppe, eine $C_3$-$C_6$-Alkinylgruppe, eine $C_1$-$C_6$-Acylgruppe oder ein Benzoylrest, wobei der aromatische Ring ein bis drei der folgenden Substituenten tragen kann: Nitro, Cyano, Halogen, $C_1$-$C_4$-Alkyl, $C_1$-$C_4$-Alkoxy, $C_1$-$C_4$-Alkylthio und/oder $C_1$-$C_4$-Halogenalkyl,

bedeuten,

sowie ihre landwirtschaftlich nutzbaren Salze und Ester von $C_1$-$C_{10}$-Carbonsäuren und anorganischen Säuren.

2. Verfahren zur Herstellung von Verbindungen der Formel I gemäß Anspruch 1, dadurch gekennzeichnet, daß man ein entsprechendes Cyclohexenon der Formel II

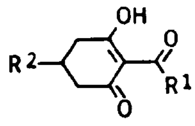

II

in an sich bekannter Weise in einem inerten organischen Lösungsmittel mit einem Hydroxylamin der Formel III

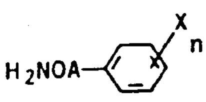

III

oder einem Salz des entsprechenden Hydroxylamins umsetzt.

3. Herbizid, enthaltend eine Verbindung der Formel I gemäß Anspruch 1 und inerte Zusatzstoffe.

4. Herbizid, enthaltend übliche Zusatzstoffe, eine Verbindung der Formel I gemäß Anspruch 1 und weitere Wirkstoffe.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonderivates der Formel I gemäß Anspruch 1 behandelt.

## Claims

1. A cyclohexenone oxime ether of the general formula I

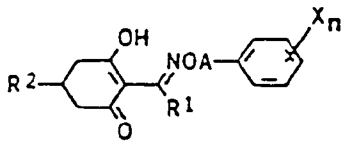

(I),

where

$R^1$ is $C_1$-$C_6$-alkyl,

A is a $C_4$-alkylene or $C_4$-alkenylene chain and these chains may carry from one to three $C_1$-$C_3$-alkyl groups and/or halogen atoms,

X is nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkoxy, carboxyl, $C_1$-$C_4$-alkoxycarbonyl, benzyloxycarbonyl and/or phenyl, and the aromatic radicals may carry from one to three of the following substituents: nitro, cyano, halogen, $C_1$-$C_4$-alkyl, $C_1$-$C_4$-alkoxy, $C_1$-$C_4$-alkylthio, $C_1$-$C_4$-haloalkyl, $C_1$-$C_4$-haloalkyloxy, carboxyl, $C_1$-$C_4$-alkoxycarbonyl and/or benzyloxycarbonyl,

n is from 0 to 3 or from 1 to 5 if X is halogen,

$R^2$ is $C_1$-$C_4$-alkoxy-$C_1$-$C_6$-alkyl or $C_1$-$C_4$-alkylthio-$C_1$-$C_6$-alkyl, $C_3$-$C_7$-cycloalkyl or $C_5$-$C_7$-cycloalkenyl, and these groups may carry from one to three of the following substituents: $C_1$-$C_4$-Alkyl, $C_1$-$C_4$-alkoxy, $C_1$-$C_4$-alkylthio, $C_1$-$C_4$-haloalkyl, hydroxyl and/or halogen,

a 5-membered saturated heterocyclic radical which contains one or two oxygen and/or sulfur atoms as heteroatoms and may carry up to three of the following groups: $C_1$-$C_4$-alkyl, $C_1$-$C_4$-alkoxy, $C_1$-$C_4$-alkylthio and/or $C_1$-$C_4$-haloalkyl,

a 6- or 7-membered heterocyclic radical which contains one or two oxygen and/or sulfur atoms and up to two double bonds, and this ring may carry from one to three of the following substituents: hydroxyl, halogen, $C_1$-$C_4$-alkyl, $C_1$-$C_4$-alkoxy, $C_1$-$C_4$-alkylthio, and/or $C_1$-$C_4$-haloalkyl,

a 5-membered heteroaromatic radical containing one or two nitrogen atoms and an oxygen or a sulfur atom, and this ring may carry from one to three of the following substituents: halogen, cyano, $C_1$-$C_4$-alkyl, $C_1$-$C_4$-alkoxy, $C_1$-$C_4$-alkylthio, $C_1$-$C_4$-haloalkyl, $C_2$-$C_6$-alkenyl, $C_2$-$C_6$-alkenyloxy, $C_2$-$C_6$-haloalkenyl and/or $C_1$-$C_4$-alkoxy-$C_1$-$C_4$-alkyl, phenyl or pyridyl and these groups may carry from one to three of the following substituents: halogen, nitro, cyano, $C_1$-$C_4$-alkyl, $C_1$-$C_4$-alkoxy, $C_1$-$C_4$-alkylthio, $C_1$-$C_4$-haloalkyl, $C_3$-$C_6$-alkenyloxy, $C_3$-$C_6$-alkynyloxy and/or -$NR^3R^4$, where

$R^3$ is hydrogen, $C_1$-$C_4$-alkyl, $C_3$-$C_6$-alkenyl or $C_3$-$C_6$-alkynyl, and $R^4$ is hydrogen, $C_1$-$C_4$-alkyl, $C_3$-$C_6$-alkenyl, $C_3$-$C_6$-alkynyl, $C_1$-$C_6$-acyl or benzoyl, and the aromatic ring may carry from one to three of the following substituents: nitro, cyano, halogen, $C_1$-$C_4$-alkyl, $C_1$-$C_4$-alkoxy, $C_1$-$C_4$-alkylthio and/or $C_1$-$C_4$-haloalkyl,

and its agriculturally useful salts and esters of $C_1$-$C_{10}$-carboxylic acids and inorganic acids.

2. A process for preparing a compound of the formula I as claimed in claim 1, wherein a corresponding cyclohexenone of the formula II

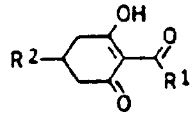

II

is reacted in conventional manner in an inert organic solvent with a hydroxylamine of the formula III

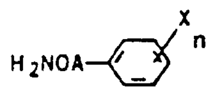

III

or a salt of the corresponding hydroxylamine.

3. A herbicide containing a compound of the formula I as claimed in claim 1 and inert additives.

4. A herbicide containing conventional additives, a compound of the formula I as claimed in claim 1 and further active ingredients.

5. A method for controlling the growth of undesirable plants, wherein the undesirable plants and/or their habitat are treated with a herbicidally effective amount of a cyclohexenone derivative of the formula I as claimed in claim 1.

## Revendications

1. Ethers de cyclohexénonoximes de formule générale I

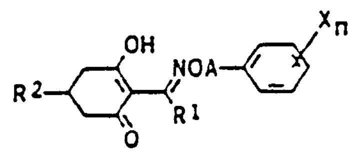

(I)

dans laquelle les symboles ont les significations suivantes :

$R^1$ représente un groupe alkyle en C1-C6,

A représente une chaîne alkylène en C4 ou alcénylène en C4, ces chaînes pouvant porter de un à trois groupes alkyle en C1-C3 et/ou atomes d'halogènes,

X représente un groupe nitro, cyano, un halogène, un groupe alkyle en C1-C4, alcoxy en C1-C4, alkylthio en C1-C4, halogénoalkyle en C1-C4, halogénoalcoxy en C1-C4, carboxyle, (alcoxy en C1-C4)-carbonyle, benzyloxycarbonyle et/ou phényle, les radicaux aromatiques pouvant porter de 1 à 3 des substituants suivants : les groupes nitro, cyano, les halogènes, les groupes alkyle en C1-C4, alcoxy en C1-C4, alkylthio en C1-C4, halogénoalkyle en C1-C4, halogénoalyloxy en C1-C4, carboxyle, (alcoxy en C1-C4)-carbonyle et/ou benzyloxycarbonyle ;

n est un nombre allant de 0 à 3 ou de 1 à 5 lorsque X représente un halogène,

$R^2$ représente un groupe (alcoxy en C1-C4)-alkyle en C1-C6 ou (alkylthio en C1-C4)-alkyle en C1-C6 ;

un groupe cycloalkyle en C3-C7 ou cycloalcényle en C5-C7, ces groupes pouvant porter de 1 à 3 des substituants suivants : les groupes alkyle en C1-C4, alcoxy en C1-C4, alkylthio en C1-C4, halogénoalkyle en C1-C4, hydroxy et/ou les halogènes ;

un hétérocycle saturé à 5 chaînons contenant 1 ou 2 atomes d'oxygène et/ou de soufre en tant qu'hétéroatomes et qui peut porter jusqu'à 3 des groupes suivants : alkyle en C1-C4, alcoxy en C1-C4, alkylthio en C1-C4 et/ou halogénoalkyle en C1-C4 ;

un hétérocycle à 6 ou 7 chaînons contenant un ou deux atomes d'oxygène et/ou de soufre et jusqu'à deux doubles liaisons, ce cycle pouvant porter 1 à 3 des substituants suivants : les groupes hydroxy, les halogènes, les groupes alkyle en C1-C4, alcoxy en C1-C4, alkylthio en C1-C4 et/ou halogénoalkyle en C1-C4,

un groupe hétéroaromatique à 5 chaînons contenant 1 à 2 atomes d'azote et un atome d'oxygène ou un atome de soufre, ce cycle pouvant porter 1 à 3 des substituants suivants : les halogènes, les groupes cyano, alkyle en C1-C4, alcoxy en C1-C4, alkylthio en C1-C4, halogénoalkyle en C1-C4, alcényle en C2-C6, alcényloxy en C2-C6, halogénoalcényle en C2-C6 et/ou (alcoxy en C1-C4)-alkyle en C1-C4 ;

un groupe phényle ou un groupe pyridyle, ces groupes pouvant porter 1 à 3 des substituants suivants : les halogènes, les groupes nitro, cyano, alkyle en C1-C4, alcoxy en C1-C4, alkylthio en C1-C4, halogénoalkyle en C1-C4, alcényloxy en C3-C6, alcynyloxy en C3-C6 et/ou $-NR^3R^4$ dans lequel

$R^3$ représente l'hydrogène, un groupe alkyle en C1-C4, alcényle en C3-C6 ou alcynyle en C3-C6 et

$R^4$ représente l'hydrogène, un groupe alkyle en C1-C4, alcényle en C3-C6, alcynyle en C3-C6, acyle en C1-C6 ou benzoyle, le cycle aromatique pouvant porter 1 à 3 des substituants suivants : les groupes nitro, cyano, les halogènes, les groupes alkyle en C1-C4, alcoxy en C1-C4, alkylthio en C1-C4 et/ou ha-

logènoalkyle en C1-C4, et leurs sels et esters d'acides carboxyliques en C1-C10 et d'acides minéraux, convenant pour des applications agricoles.

2. Procédé de préparation des composés de formule I de la revendication 1, caractérisé en ce que l'on fait réagir une cyclohexénone correspondante de formule II

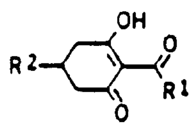

II

de manière connue en soi, dans un solvant organique inerte, avec une hydroxylamine de formule III

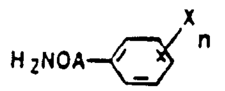

III

ou un sel de cette hydroxylamine.

3. Produit herbicide contenant un composé de formule I de la revendication 1 et des additifs inertes.

4. Produit herbicide contenant des additifs usuels, un composé de formule I de la revendication 1, et d'autres substances actives.

5. Procédé pour combattre les croissances de végétaux indésirables, caractérisé en ce que l'on traite les végétaux indésirables et/ou leur habitat par une quantité herbicide efficace d'un dérivé de cyclohexénone de formule I de la revendication 1.